# FASCICULE DE BREVET EUROPEEN

(11) **EP 2 691 154 B1**
(45) Date de publication et mention de la délivrance du brevet: **29.04.2015**
(21) Numéro de dépôt: 12714789.0
(22) Date de dépôt: 15.03.2012
(51) Int. Cl.: A61N 7/02

(54) **SONDE DE THERAPIE POUR LE TRAITEMENT DE TISSUS PAR L'INTERMEDIAIRE D'ONDES ULTRASONORES FOCALISEES CROISEES**
THERAPEUTISCHE SONDE ZUR BEHANDLUNG VON GEWEBE MIT SICH ÜBERSCHNEIDENDEN FOKUSSIERTEN ULTRASCHALLWELLEN
THERAPEUTIC PROBE FOR TREATING TISSUE USING FOCUSED INTERSECTING ULTRASONIC WAVES

(30) Priorité: 29.03.2011 FR 1152600
(43) Date de publication de la demande: 05.02.2014
(73) Titulaire: EDAP TMS France, 69120 Vaulx-en-Velin (FR); INSERM - Institut National de la Santé et de la Recherche Médicale, 75654 Paris Cedex 13 (FR)
(72) Inventeur: MELODELIMA, David, 38300 Ruy (FR); VINCENOT, Jérémy, 69420 Les Haies (FR); BLANC, Emmanuel, 69370 Saint Didier au Mont D'or (FR); CHAPELON, Jean-Yves, 69100 Villeurbanne (FR)
(74) Mandataire: Thibault, Jean-Marc
(86) Numéro de dépôt international: PCT/FR2012/050546
(87) Numéro de publication internationale: WO 2012/131213

(56) Documents cités:
- WO-A1-2006/021651
- WO-A1-2010/029556
- US-A1- 2004 030 268
- US-A1- 2009 230 823
- M. William Apoutou N'djin: "Transducteur torique à Ultrasons Focalisés de Haute Intensité pour générer des ablations volumineuses", , 17 décembre 2008 (2008-12-17), XP055009820, Extrait de l'Internet: URL:http://u556.lyon.inserm.fr/theses/pdf/ ndjin.pdf [extrait le 2011-10-18]

## Description

La présente invention concerne le domaine technique des appareils ou des dispositifs comportant une sonde ultrasonore formée par une pluralité d'éléments transducteurs ultrasonores, adaptée pour émettre des ultrasons focalisés de haute intensité (HIFU).

L'objet de la présente invention trouve des applications particulièrement avantageuses dans le domaine des traitements thérapeutiques par des ondes ultrasonores focalisées.

Il est connu que la thérapie par ultrasons focalisés permet de créer des lésions biologiques dans les tissus résultant d'une combinaison des effets thermiques et de l'activité de cavitation acoustique. La forme de ces lésions tissulaires est issue directement de la forme de la surface d'émission de la sonde ultrasonore utilisée.

Par exemple, la focalisation géométrique naturelle d'un transducteur ultrasonore HIFU sphérique classique est ponctuelle mais du fait des aberrations géométriques, la zone focale est de forme ellipsoïdale. De même, un transducteur HIFU de forme torique conduit à l'obtention d'une zone de focalisation en forme d'anneau ou de couronne.

Quelle que soit la forme du transducteur ultrasonore, il est à considérer que plus le transducteur ultrasonore est focalisé, meilleure est la résolution spatiale de la zone de focalisation et de la lésion biologique. Il en découle une préservation des tissus voisins de ceux situés dans la zone de focalisation, au dépend d'un volume de lésions réduit et donc d'une durée de traitement plus longue.

Pour réduire la durée de traitement, il convient d'augmenter le volume de la lésion biologique issue de l'application de chacune des impulsions ultrasonores. Pour cela, différentes solutions sont envisageables. Par exemple, il peut être prévu d'augmenter le volume focal par réduction de l'ouverture du transducteur ultrasonore. Toutefois, dans un tel cas, le gradient de pression acoustique sur le trajet de l'onde ultrasonore est également réduit et les zones des tissus intermédiaires entre le transducteur et la zone focale sont alors exposées à un échauffement plus important.

Une autre solution consiste à augmenter le volume focal par réduction de la fréquence de fonctionnement. Cependant, la réduction de la fréquence des ondes ultrasonores produit également une réduction de l'énergie absorbée et donc du dépôt de chaleur dans les tissus.

Il peut être également envisagé d'augmenter la puissance acoustique émise par le transducteur ultrasonore. Toutefois, les zones des tissus situées entre la zone focale et le transducteur se trouvent alors exposées à des puissances acoustiques plus intenses susceptibles de conduire à des lésions en dehors de la zone ciblée.

Le brevet US 5 873 845 propose une autre solution consistant à placer une lentille réfractante devant le transducteur ultrasonore en vue d'élargir la zone de focalisation. Si cette solution est simple et peu coûteuse, elle offre des performances limitées et la lentille absorbe une partie de l'énergie acoustique émise.

Le brevet EP 0 214 782 décrit une autre solution relative à un transducteur ultrasonore équipé d'une lentille permettant d'obtenir une focalisation annulaire à partir d'un transducteur de géométrie sphérique. Cette construction particulière permet d'élargir la zone focale à la taille de l'anneau mais fait également apparaître une zone de recouvrement située sur l'axe central du transducteur issu du croisement des faisceaux ultrasonores au-delà du plan de focalisation du transducteur. Ce document prévoit un système pour réduire le champ de pression dans cette zone de recouvrement afin de s'affranchir du risque de lésions secondaires.

Le document XP 55009820 décrit une sonde de thérapie comportant une face d'émission de révolution engendrée par la rotation autour d'un axe de symétrie, d'un segment de courbe concave avec le centre de courbure qui se trouve à distance de l'axe de symétrie. Cette face d'émission présente dans un plan de profil, deux segments de courbe concave symétriques, par rapport à l'axe de symétrie, avec chaque segment de courbe concave possédant un axe acoustique passant par le centre de courbure et le milieu du segment de courbe concave.

Un tel transducteur permet d'obtenir d'une part, un anneau de focalisation des ondes ultrasonores délimitées par le plan focal et d'autre part, une zone de croisement des faisceaux ultrasonores. Comme il ressort clairement de ce document, la zone de croisement des faisceaux ultrasonores qui correspond au pic secondaire de pression est située derrière le plan focal.

Le document US 2009/230823 décrit une sonde de thérapie comportant un transducteur présentant une face d'émission sphérique comportant des séries d'émetteurs ultrasonores susceptibles d'être sollicitées de manière indépendante. Les groupes de transducteurs sont dirigés pour viser un point focal commun. Dans la mesure où les transducteurs sont cofocaux, les axes des transducteurs acoustiques sont focalisés en un seul point.

La présente invention vise à remédier aux inconvénients des diverses solutions techniques antérieures en proposant une nouvelle sonde de thérapie adaptée pour permettre d'obtenir un volume de lésions biologiques relativement important tout en préservant les zones biologiques voisines ne devant pas être traitées par les ondes ultrasonores.

Pour atteindre un tel objectif, la sonde de thérapie pour le traitement de tissus par l'intermédiaire d'ondes ultrasonores focalisées, comporte un transducteur formé par une pluralité d'émetteurs ultrasonores répartis sur une face d'émission pour focaliser les ondes ultrasonores dans une première zone focale s'établissant dans un plan focal, la face d'émission présentant dans un plan de profil, deux segments de courbe concave de longueur finie, symétriques par rapport à un plan de symétrie ou un axe de symétrie, chaque segment de courbe concave possède un axe acoustique passant par le centre de courbure et le milieu dudit segment de courbe concave, cette face d'émission étant engendrée soit par la rotation de l'un des segments de courbe concave autour de l'axe de symétrie soit par la translation des deux segments de courbe selon une direction perpendiculaire au plan de profil contenant lesdits segments de courbe.

Selon l'invention :
- dans le plan de profil, les deux segments de courbe concave suivent des arcs d'un premier et deuxième cercles non con-coïncidents qui se coupent, l'un des deux segments de courbe concave suivant l'arc du premier cercle avec cet arc qui est situé à l'intérieur du deuxième cercle, tandis que l'autre des deux segments de courbe concave suivant l'arc du deuxième cercle, avec l'arc étant situé à l'intérieur du premier cercle,
- chaque segment de courbe présente un axe acoustique coupant l'axe de symétrie ou le plan de symétrie entre la première zone focale et la face d'émission, les axes acoustiques étant écartés l'un de l'autre, au niveau de la première zone focale, d'une distance d'écartement comprise entre 1 et 50 mm, de manière que les faisceaux de la face d'émission se croisent pour créer une deuxième zone focale qui est localisée et située entre la première zone focale et la face d'émission, et à distance du plan focal,
- la face d'émission comporte un bord interne dont les parties symétriques s'étendant de part et d'autre de l'axe de symétrie ou du plan de symétrie sont écartées l'une de l'autre d'une distance interne comprise entre 10 et 120 mm permettant le positionnement de la deuxième zone focale à distance de la face d'émission.

De plus, la sonde de thérapie selon l'invention peut présenter en outre en combinaison, l'une et/ou l'autre des caractéristiques additionnelles suivantes :
- la face d'émission comporte un bord externe dont les parties symétriques s'étendant de part et d'autre de l'axe de symétrie du plan de symétrie sont écartées l'une de l'autre d'une distance externe comprise entre 30 et 300 mm,
- le bord interne délimite dans la face d'émission, un logement pour le montage d'une sonde d'imagerie ultrasonore,
- chaque segment de courbe concave est un segment d'arc de cercle concave,
- la face d'émission est issue d'une géométrie torique engendrée par la rotation d'un des segments de courbe concave autour de l'axe de symétrie de sorte que la première zone focale possède une forme en couronne,
- la face d'émission est tronquée de manière symétrique par rapport à l'axe de symétrie,
- la face d'émission est issue d'une géométrie cylindrique engendrée par la translation selon une longueur limitée, des deux segments de courbe selon une direction perpendiculaire au plan de profil contenant lesdits segments de courbe de sorte que la première zone focale possède une forme linéaire double.

Un autre objet de l'invention vise un appareil de thérapie pour le traitement de tissus par l'émission d'ondes ultrasonores focalisées, comportant une sonde de thérapie dont les émetteurs ultrasonores sont activés par des signaux délivrés par un générateur de signal faisant partie d'un circuit de commande, de manière à assurer la focalisation des ondes ultrasonores dans une première zone focale et à obtenir une deuxième zone focale localisée et située entre la première zone focale et la face d'émission.

Diverses autres caractéristiques ressortent de la description faite ci-dessous en référence aux dessins annexés qui montrent, à titre d'exemples non limitatifs, des formes de réalisation de l'objet de l'invention.
La Figure 1 est un schéma de principe d'une sonde de thérapie conforme à l'invention.
La Figure 2 est une vue en perspective d'une sonde de thérapie torique conforme à l'invention.
La **Figure 2A** est une vue en coupe élévation de la sonde de thérapie torique conforme à l'invention, illustrée à la **Fig. 2****.**
La **Figure 3** est une vue en perspective d'une sonde de thérapie torique tronquée.
La **Figure 4** est une vue en perspective d'une sonde de thérapie composée de deux portions de cylindre.
La **Figure 5** est une vue en coupe élévation de la sonde de thérapie illustrée à la **Fig. 4****.**

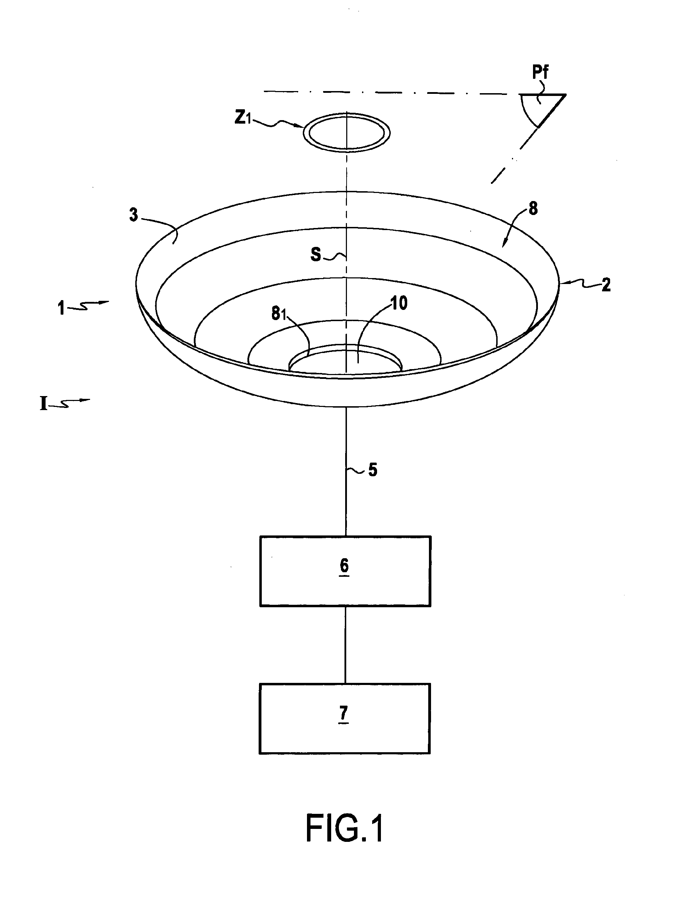

Tel que cela ressort plus précisément de la **Fig. 1****,** l'objet de l'invention concerne une sonde de thérapie **1** faisant partie d'un appareil de thérapie **I** au sens général adaptée pour réaliser le traitement de tissus d'un être vivant par l'intermédiaire d'ultrasons focalisés de haute intensité (HIFU). La sonde de thérapie **1** comporte notamment un transducteur **2** comportant un ou plusieurs émetteurs ultrasonores **3** tels que par exemple des éléments piézoélectriques. Ces émetteurs ultrasonores **3** sont reliés par l'intermédiaire de câbles coaxiaux **5** via, un étage amplificateur **6** à un circuit de commande **7** délivrant des signaux pour activer les émetteurs ultrasonores **3.** Le circuit de commande **7** n'est pas décrit plus précisément car sa réalisation fait partie des connaissances techniques de l'homme du métier. Ce circuit de commande **7** comporte ainsi classiquement un générateur de signal piloté qui est relié aux émetteurs ultrasonores par l'intermédiaire de l'étage amplificateur **6.**

Le transducteur **2** présente une face **8** d'émission d'ondes ultrasonores focalisées dans une première zone focale **Z₁** dont la géométrie dépend de la forme de la face d'émission du transducteur ultrasonore.

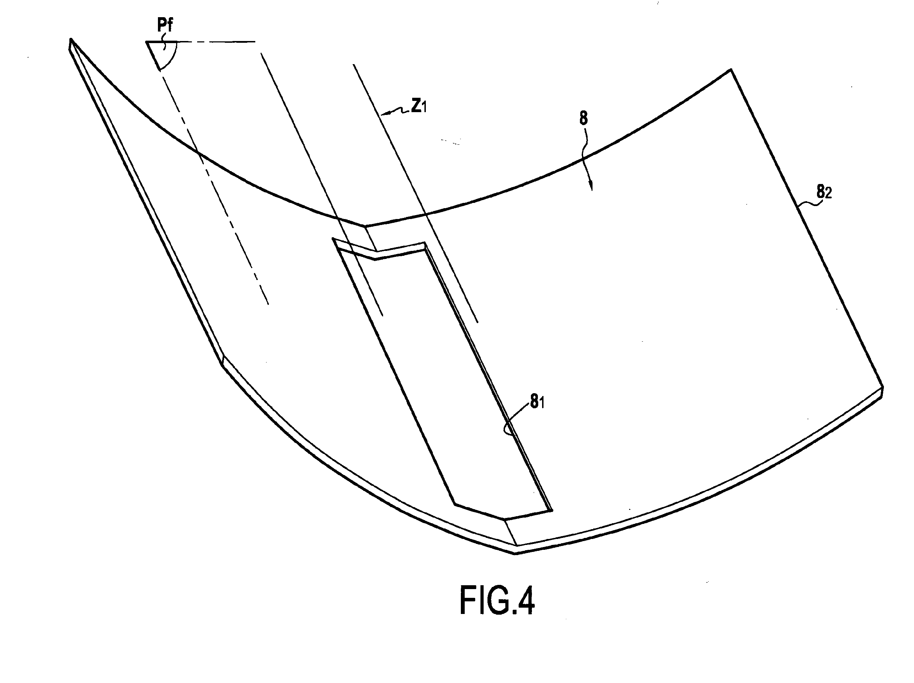

Dans l'exemple illustré aux **Fig. 1** à **3****,** la première zone focale **Z₁** présente une forme d'anneau ou de couronne s'établissant dans un plan focal **Pf** alors que dans l'exemple illustré à la **Fig. 4****,** la première zone focale **Z₁** possède une forme linéaire double s'établissant dans un plan focal **Pf.**

Dans l'exemple de réalisation illustré aux **Fig. 1** à **3****,** la face d'émission **8** du transducteur ultrasonore **2** est une surface de révolution engendrée par la rotation, autour d'un axe de symétrie **S,** d'un segment de courbe concave **s₁** de longueur finie **l** et présentant un centre de courbure **C** situé dans le plan focal **Pf.** Le centre de courbure **C** est situé du côté opposé audit segment de courbe **s₁** par rapport à l'axe de symétrie **S.** Ainsi, l'axe acoustique ou de focalisation **a** passant par le centre de courbure **C** et le milieu du segment de courbe concave **s₁** coupe l'axe de symétrie **S** entre le plan focal **Pf** et la face d'émission **8.** La géométrie de cette face d'émission **8** peut être considérée comme issue d'une géométrie torique croisée.

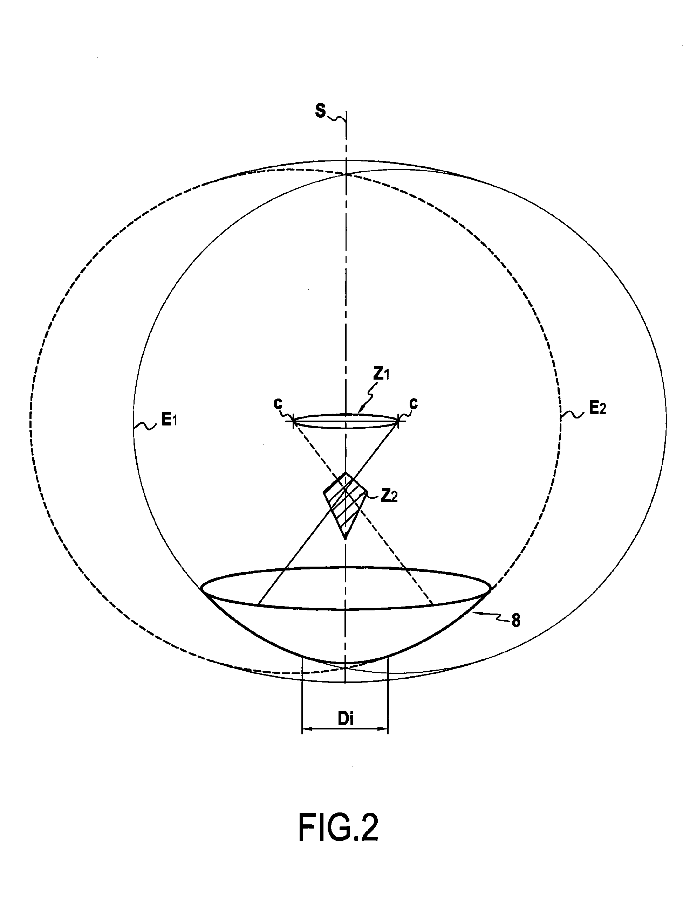

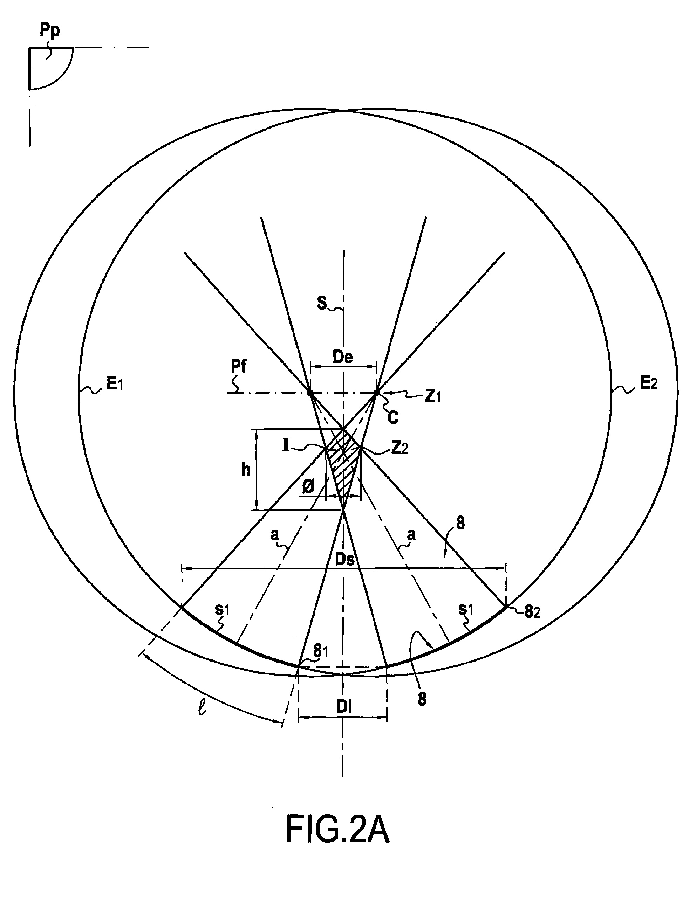

Comme cela apparaît clairement aux **Fig. 2** et **2A****,** cette face d'émission **8** présente dans le plan de profil **Pp,** deux segments de courbe concave **s₁** symétrique par rapport à l'axe de symétrie **S.** Ainsi, chaque segment concave **s₁** de la face d'émission **8** focalise les ondes ultrasonores en un point de la première zone focale **Z₁** situé au-delà de l'axe de symétrie **S** par rapport à la face d'émission **8.** Aussi, le segment de courbe **s₁** situé à droite sur la **Fig. 2****,** focalise l'onde ultrasonore sur le côté gauche de la première zone focale **Z₁** et réciproquement.

Tel que cela ressort des **Fig. 2** et **2A****,** la face d'émission **8** comporte dans le plan de profil **Pp,** deux segments de courbes concaves **s₁** qui suivent les arcs d'un premier cercle **E₁** possédant un centre **C** et d'un deuxième cercle **E₂** possédant un autre centre **C** différent du centre du premier cercle **E₁.** Les premier et deuxième cercles **E₁, E₂** ne coïncident pas mais se coupent entre eux. L'un des deux segments de courbe concave **s₁** (à gauche sur les **Fig. 2** et **2A****),** suit l'arc du premier cercle **E₁,** cet arc du premier cercle **E₁** étant situé à l'intérieur du deuxième cercle **E₂.** De manière similaire, l'autre des deux segments concaves **s₁** (à droite sur les **Fig. 2** et **2A****)** suit l'arc du deuxième cercle **E₂,** cet arc du deuxième cercle **E₂** étant situé à l'intérieur du premier cercle **E₁.**

Il ressort de la description qui précède que la première zone focale **Z₁** présente ainsi une forme annulaire avec un diamètre d'écartement **Dₑ.** Selon une caractéristique avantageuse, les axes acoustiques **a.** des deux segments de courbe **s₁** symétriques sont écartés l'un de l'autre au niveau du plan de focalisation **Pf** d'une distance d'écartement **Dₑ** comprise entre 1 et 50 mm.

Il est à noter que dans ces conditions, pour deux parties de la face d'émission **8,** situées de manière symétrique par rapport à l'axe de symétrie **S,** les axes acoustiques **a** correspondants coupent l'axe de symétrie **S** en un point d'intersection commun **I** situé entre la première zone focale **Z₁** et la face d'émission **8.** Ainsi, les faisceaux de la face d'émission **8** se croisent pour former une deuxième zone focale **Z₂** qui est localisée et située entre la première zone focale **Z₁** et la face d'émission **8.** Cette deuxième zone focale **Z₂** qui correspond à une zone de recouvrement des faisceaux ultrasonores présente deux dimensions caractéristiques à savoir son diamètre 0 et sa longueur h **(****Fig. 2****).**

Selon une autre caractéristique qui apparaît plus précisément à la **Fig. 1****,** la face d'émission **8** présente une découpe ou une ouverture **10** centrée sur l'axe de symétrie **S.** La face d'émission **8** présente ainsi un bord interne **8₁** délimitant ainsi la découpe **10.** Les parties du bord interne **8₁** symétriques par rapport à l'axe de symétrie **S** sont écartées l'une de l'autre d'une distance interne **Dᵢ** comprise entre 10 et 120 mm. En d'autres termes, les parties du bord interne **8₁** de deux segments de courbe symétriques par rapport à l'axe de symétrie **S** sont écartées l'une de l'autre de la distance interne **Dᵢ** permettant de faire varier le diamètre 0 ainsi que de positionner la deuxième zone focale **Z₂** à distance de la face d'émission **8,** en faisant varier sa longueur h.

Tel que cela ressort clairement de la **Fig. 2****,** la deuxième zone focale **Z₂** est localisée en-deçà du plan focal **Pf** permettant ainsi de ramener l'énergie acoustique entre le plan focal **Pf** du transducteur et sa face d'émission **8.** Cette deuxième zone focale **Z₂** qui correspond à la zone de recouvrement des ondes ultrasonores est avantageusement utilisée pour créer une lésion biologique volumineuse.

Cette technique va à l'encontre de la démarche classique qui préconise de déposer l'énergie acoustique à distance du transducteur et en particulier au-delà du plan focal **Pf** de manière à préserver au mieux les structures biologiques intermédiaires situées entre le plan focal **Pf** et le transducteur **2.** Toutefois, l'intérêt de cette invention réside dans l'exploitation de la zone de recouvrement des faisceaux située en deçà du plan focal **Pf,** en tant que zone de traitement, et dans la suppression du croisement des faisceaux ultrasonores au-delà du plan focal qui entraîne des lésions secondaires non maîtrisées avec des géométries très pointues non pertinentes par rapport à la géométrie des tissus à traiter. La présente invention permet de maîtriser la géométrie des lésions réalisées par la combinaison des première **Z₁** et deuxième **Z₂** zones focales.

Les dimensions 0 et h de la deuxième zone focale **Z₂** peuvent également être réglées à partir de la distance interne **Dᵢ** séparant les parties opposées du bord interne **8₁.** A cet égard, il est à noter que le bord interne **8₁** délimite avantageusement un logement pour le montage d'une sonde d'imagerie ultrasonore. La distance externe **Dₛ** permet de localiser la deuxième zone focale **Z₂** à distance du plan focal **P_{f}.** En d'autres termes, la deuxième zone focale **Z₂** ne touche pas le plan focal **P_{f}.** Selon une variante de réalisation, la deuxième zone focale **Z₂** et la première zone focal **Z₁** sont distinctes l'une de l'autre c'est-à-dire qu'elles sont séparées.

Les dimensions de la deuxième zone focale **Z₂** peuvent également être maîtrisées à partir notamment de la distance externe **Ds** prise entre les parties symétriques par rapport à l'axe de symétrie **S,** d'un bord externe **8₂** délimitant la périphérie de la face d'émission **8.** Avantageusement, les parties symétriques du bord externe **8₂** s'étendant de part et d'autre de l'axe de symétrie sont écartées l'une de l'autre d'une distance externe **D_{S}** comprise entre 30 et 300 mm.

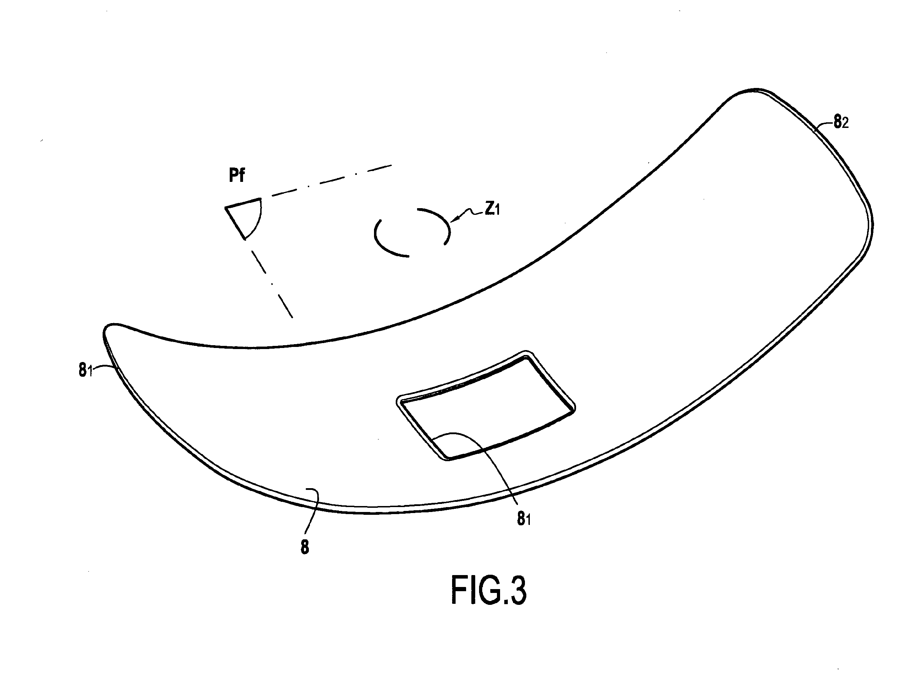

Selon une forme avantageuse de réalisation de la variante illustrée aux **Fig. 1** et **2****,** chaque segment de courbe concave **s₁** est un segment d'arc de cercle concave. Dans l'exemple illustré aux **Fig. 1** et **2****,** la face d'émission **8** est une surface de révolution complète comportant, par exemple, une série d'éléments transducteurs ultrasonores **3** montés de manière concentrique les uns par rapport aux autres et par rapport à l'axe de symétrie **S.** Bien entendu, il peut être envisagé comme illustré à la **Fig. 3****,** que la face d'émission **8** soit tronquée de manière symétrique par rapport à l'axe de symétrie **S** afin que le transducteur se limite à une portion d'un tore croisé présentant une largeur comprise, par exemple, entre 5 et 250 mm. Selon cet exemple de réalisation, le transducteur **2** comporte une série d'éléments transducteurs ultrasonores **3** montés de manière concentrique les uns par rapport aux autres et par rapport à l'axe de symétrie **S** de sorte qu'ils présentent chacun sous la forme d'un segment d'anneau ou de couronne.

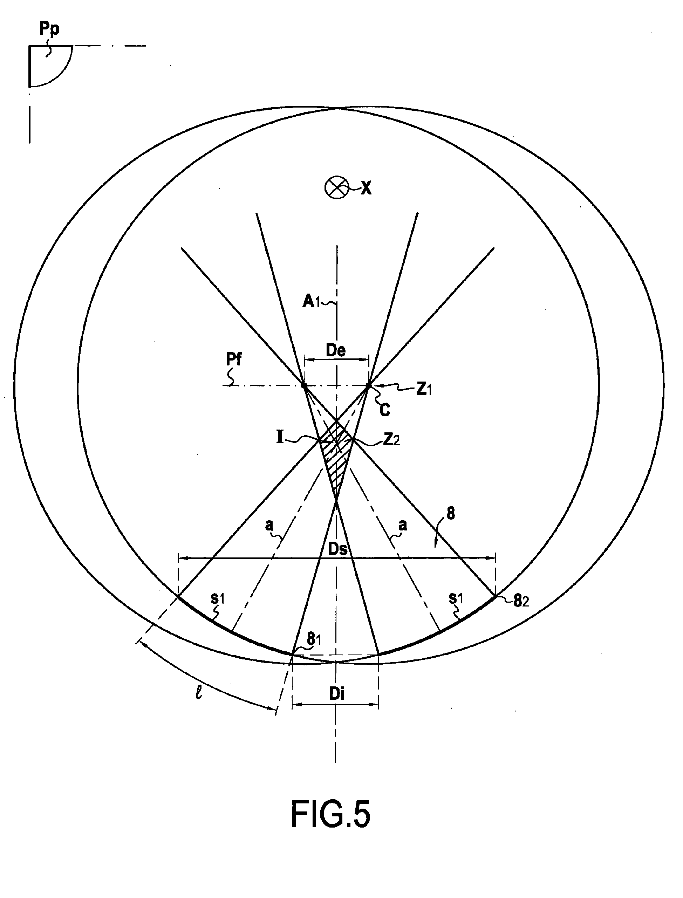

La **Fig. 4** et **5** illustrent une autre variante de réalisation de la face d'émission **8** dans laquelle cette face d'émission **8** est engendrée par la translation des deux segments de courbe **s₁** symétriques, selon une direction **X** perpendiculaire au plan de profil **Pp** contenant les deux segments de courbe **s₁**. Cette translation est réalisée selon la direction **X** selon une longueur limitée adaptée à la taille souhaitée pour la face d'émission **8.** Selon cette variante de réalisation, la face d'émission **8** présente une forme pseudo-cylindrique, tubulaire ou composée de deux portions de cylindre alors que dans l'exemple illustré aux **Fig. 1** et **2****,** la face d'émission **8** est de révolution. Cependant, tel que cela ressort de la comparaison des **Fig. 2** et **5****,** la face d'émission **8** illustrée aux Fig. **4****,** **5** possède en profil, les mêmes caractéristiques géométriques que celles décrites aux **Fig. 1** et **2****.**

Chaque segment de courbe **s₁** est de forme concave et de longueur finie. Les deux segments de courbe **s₁** sont symétriques par rapport à un plan de symétrie **A₁** perpendiculaire au plan de profil **Pp.** Chaque segment de courbe **s₁** présente ainsi un axe acoustique **a** coupant le plan de symétrie **A₁** entre la première zone focale **Z₁** et la face d'émission **8.** Chaque segment de courbe **s₁** de la face d'émission 8 focalise les ondes ultrasonores en un point de la première zone focale **Z₁** situé au-delà du plan de symétrie **A₁** par rapport à la face d'émission **8.** Aussi, compte tenu de la symétrie de type plan, la première zone focale **Z₁** possède une forme linéaire double. En effet, chaque partie de la face d'émission **8** focalise, au-delà du plan de symétrie **A₁,** selon un segment linéaire s'étendant dans le plan focal **Pf,** parallèlement à la direction **X.**

Les axes acoustique **a** des deux segments de courbe symétriques **s1** sont écartés au niveau de la première zone focale **Z₁** d'une distance interne **Dₑ** selon les caractéristiques décrites ci-dessus. De même, selon cette variante, il est créé une deuxième zone focale **Z₂** qui est localisée et située entre la première zone focale **Z₁** et la face d'émission **8.** Cette deuxième zone focale **Z₂** présente les mêmes caractéristiques que celles décrites en relation des **Fig. 1** et **2****.**

Dans la description qui précède, la face d'émission **8** est représentée comme faisant partie intrinsèquement du transducteur **2.** Bien entendu, il est clair que la face d'émission **8** peut être formée par le transducteur mais également par la surface d'un réflecteur, d'une lentille acoustique ou autre.

## Revendications

1. Sonde de thérapie pour le traitement de tissus par l'intermédiaire d'ondes ultrasonores focalisées, comportant un transducteur **(2)** formé par une pluralité d'émetteurs ultrasonores **(3)** répartis sur une face d'émission **(8)** pour focaliser les ondes ultrasonores dans une première zone focale **(Z₁)** s'établissant dans un plan focal **(Pf),** la face d'émission **(8)** présentant, dans un plan de profil **(Pₚ),** deux segments de courbe concave **(s₁)** de longueur finie, symétriques par rapport à un plan de symétrie **(A₁)** ou un axe de symétrie **(S),** chaque segment de courbe concave **(s₁)** possède un axe acoustique **(a)** passant par le centre de courbure **(C)** et le milieu dudit segment de courbe concave **(s₁),** cette face d'émission **(8)** étant engendrée soit par la rotation de l'un des segments de courbe concave **(s₁)** autour de l'axe de symétrie **(S)** soit par la translation des deux segments de courbe **(s₁)** selon une direction **(X)** perpendiculaire au plan de profil **(Pₚ)** contenant lesdits segments de courbe, **caractérisée en ce que** :
- dans le plan de profil **(Pₚ),** les deux segments de courbe concave **(s₁)** suivent des arcs d'un premier **(E₁)** et deuxième **(E₂)** cercles non con-coïncidents qui se coupent, l'un des deux segments de courbe concave **(s₁)** suivant l'arc du premier cercle, cet arc étant situé à l'intérieur du deuxième cercle, tandis que l'autre des deux segments de courbe concave **(s₁)** suit l'arc du deuxième cercle, ledit arc étant situé à l'intérieur du premier cercle,
- chaque segment de courbe **(s₁)** présente un axe acoustique **(a)** coupant l'axe de symétrie **(S)** ou le plan de symétrie **(A₁)** entre la première zone focale **(Z₁)** et la face d'émission **(8),** les axes acoustiques **(a)** étant écartés l'un de l'autre, au niveau de la première zone focale **(Z₁),** d'une distance d'écartement (**Dₑ**) comprise entre 1 et 50 mm, de manière que les faisceaux de la face d'émission **(8)** se croisent pour créer une deuxième zone focale **(Z₂)** qui est localisée et située entre la première zone focale **(Z₁)** et la face d'émission **(8),** et à distance du plan focal **(Pf),**
- la face d'émission **(8)** comporte un bord interne **(8₁)** dont les parties symétriques s'étendant de part et d'autre de l'axe de symétrie **(S)** ou du plan de symétrie **(A₁)** sont écartées l'une de l'autre d'une distance interne **(Dᵢ)** comprise entre 10 et 120 mm permettant le positionnement de la deuxième zone focale à distance de la face d'émission.

2. Sonde de thérapie selon la revendication 1, **caractérisée en ce que** la face d'émission **(8)** comporte un bord externe dont les parties symétriques s'étendant de part et d'autre de l'axe de symétrie du plan de symétrie sont écartées l'une de l'autre d'une distance externe **(Dₛ)** comprise entre 30 et 300 mm.

3. Sonde de thérapie selon la revendication 1, **caractérisée en ce que** le bord interne **(8₁)** délimite, dans la face d'émission **(8),** un logement pour le montage d'une sonde d'imagerie ultrasonore.

4. Sonde de thérapie selon l'une des revendications 1 à 3, **caractérisée en ce que** chaque segment de courbe concave **(s₁)** est un segment d'arc de cercle concave.

5. Sonde de thérapie selon l'une des revendications 1 à 4, **caractérisée en ce que** la face d'émission **(8)** est issue d'une géométrie torique engendrée par la rotation d'un des segments de courbe concave **(s₁)** autour de l'axe de symétrie **(S)** de sorte que la première zone focale **(Z₁)** possède une forme en couronne.

6. Sonde de thérapie selon la revendication 5, **caractérisée en ce que** la face d'émission **(8)** est tronquée de manière symétrique par rapport à l'axe de symétrie **(S).**

7. Sonde de thérapie selon l'une des revendications 1 à 4, **caractérisée en ce que** la face d'émission **(8)** est issue d'une géométrie cylindrique engendrée par la translation selon une longueur limitée, des deux segments de courbe selon une direction **(X)** perpendiculaire au plan de profil **(Pp)** contenant lesdits segments de courbe de sorte que la première zone focale **(Z₁)** possède une forme linéaire double.

8. Appareil de thérapie pour le traitement de tissus par l'émission d'ondes ultrasonores focalisées, **caractérisé en ce qu'**il comporte une sonde de thérapie **(1)** conforme à l'une des revendications 1 à 7 dont les émetteurs ultrasonores **(3)** sont activés par des signaux délivrés par un générateur de signal faisant partie d'un circuit de commande **(7),** de manière à assurer la focalisation des ondes ultrasonores dans une première zone focale **(Z₁)** et à obtenir une deuxième zone focale **(Z₂)** localisée et située entre la première zone focale **(Z₁)** et la face d'émission **(8).**

## Patentansprüche

1. Therapeutische Sonde zur Behandlung von Gewebe mit fokussierten Ultraschallwellen, umfassend einen Wandler (2), der von einer Vielzahl von Ultraschallsendern (3) gebildet ist, die auf einer Sendefläche (8) verteilt sind, um die Ultraschallwellen in einer ersten Fokalzone (Z₁), die sich in einer Fokalebene (Pf) bildet, zu fokussieren, wobei die Sendefläche (8) in einer Profilebene (Pₚ) zwei konkav gekrümmte Segmente (s₁) von endlicher Länge aufweist, die in Bezug zu einer Symmetrieebene (A₁) oder einer Symmetrieachse (S) symmetrisch sind, wobei jedes konkav gekrümmte Segment (s₁) eine akustische Achse (a) besitzt, die durch den Krümmungsmittelpunkt (C) und die Mitte des konkav gekrümmten Segments (s₁) verläuft, wobei diese Sendefläche (8) entweder durch die Drehung eines der konkav gekrümmten Segmente (s₁) um die Symmetrieachse (S) oder durch die Translation der beiden gekrümmten Segmente (s₁) entlang einer Richtung (X) senkrecht zur Profilebene (Pₚ), die die gekrümmten Segmente enthält, erzeugt wird, **dadurch gekennzeichnet, dass**:
- in der Profilebene (Pₚ) die beiden konkav gekrümmten Segmente (s₁) Bögen von ersten (E₁) und zweiten (E₂) nicht zusammenfallenden Kreisen, die sich schneiden, folgen, wobei eines der beiden konkav gekrümmten Segmente (s₁) dem Bogen des ersten Kreises folgt, wobei dieser Bogen innerhalb des zweiten Kreises angeordnet ist, während das andere der beiden konkav gekrümmten Segmente (s₁) dem Bogen des zweiten Kreises folgt, wobei der Bogen innerhalb des ersten Kreises angeordnet ist,
- jedes gekrümmte Segment (s₁) eine akustische Achse (a) aufweist, die die Symmetrieachse (S) oder die Symmetrieebene (A₁) zwischen der ersten Fokalzone (Z₁) und der Sendefläche (8) schneidet, wobei die akustischen Achsen (a) im Bereich der ersten Fokalzone (Z₁) um einen Entfernungsabstand (Dₑ) zwischen 1 und 50 mm voneinander entfernt sind, so dass sich die Strahlen der Sendefläche (8) überschneiden, um eine zweite Fokalzone (Z₂) zu erzeugen, die zwischen der ersten Fokalzone (Z₁) und der Sendefläche (8) und in einem Abstand zu der Fokalebene (Pf) lokalisiert und angeordnet ist,
- die Sendefläche (8) einen Innenrand (8₁) umfasst, dessen symmetrische Teile sich beiderseits der Symmetrieachse (S) oder der Symmetrieebene (A₁) erstrecken und voneinander um einen Innenabstand (Dᵢ) zwischen 10 und 120 mm entfernt sind, wodurch die Positionierung der zweiten Fokalzone in einem Abstand zu der Sendefläche möglich ist.

2. Therapeutische Sonde nach Anspruch 1, **dadurch gekennzeichnet, dass** die Sendefläche (8) einen Außenrand umfasst, dessen symmetrische Teile sich beiderseits der Symmetrieachse der Symmetrieebene erstrecken und voneinander um einen Außenabstand (Dₛ) zwischen 30 und 300 mm entfernt sind.

3. Therapeutische Sonde nach Anspruch 1, **dadurch gekennzeichnet, dass** der Innenrand (8₁) in der Sendefläche (8) eine Lagerung für die Montage einer Ultraschallbildgebungssonde begrenzt.

4. Therapeutische Sonde nach einem der Ansprüche 1 bis 3, **dadurch gekennzeichnet, dass** jedes konkav gekrümmte Segment (s₁) ein konkaves Kreisbogensegment ist.

5. Therapeutische Sonde nach einem der Ansprüche 1 bis 4, **dadurch gekennzeichnet, dass** die Sendefläche (8) von einer Ringgeometrie stammt, die durch die Drehung eines der konkav gekrümmten Segmente (s₁) um die Symmetrieachse (S) erzeugt wird, so dass die erste Fokalzone (Z₁) eine Kranzform besitzt.

6. Therapeutische Sonde nach Anspruch 5, **dadurch gekennzeichnet, dass** die Sendefläche (8) symmetrisch in Bezug zur Symmetrieachse (S) abgeschnitten ist.

7. Therapeutische Sonde nach einem der Ansprüche 1 bis 4, **dadurch gekennzeichnet, dass** die Sendefläche (8) von einer zylindrischen Geometrie stammt, die durch die Translation entlang einer begrenzten Länge der beiden gekrümmten Segmente entlang einer Richtung (X) senkrecht auf die Profilebene (Pp), die die gekrümmten Segmente enthält, erzeugt wird, so dass die erste Fokalzone (Z₁) eine doppelte lineare Form besitzt.

8. Therapeutisches Gerät zur Behandlung von Gewebe durch Entsenden von fokussierten Ultraschallwellen, **dadurch gekennzeichnet, dass** es eine therapeutische Sonde (1) nach einem der Ansprüche 1 bis 7 umfasst, deren Ultraschallsender (3) durch Signale aktiviert werden, die von einem Signalerzeuger, der Teil der Steuerschaltung (7) ist, geliefert werden, um die Fokussierung der Ultraschallwellen in einer ersten Fokalzone (Z₁) zu gewährleisten und eine zweite Fokalzone (Z₂) zu erhalten, die zwischen der ersten Fokalzone (Z₁) und der Sendefläche (8) lokalisiert und angeordnet ist.

## Claims

1. A therapeutic probe for treating tissue using focused ultrasonic waves, including a transducer **(2)** formed by a plurality of ultrasonic sources **(3)** distributed over an emission surface **(8)** to focus the ultrasonic waves into a first focal area **(Z₁)** established in a focal plane **(Pf),** the emission surface **(8)** having, within a profile plane **(Pₚ),** two concave curve segments **(s₁)** which have a finite length, and which are symmetrical relative to a plane of symmetry **(A₁)** or to an axis of symmetry **(S),** each concave curve segment **(s₁)** has an acoustic axis **(a)** passing through the center of curvature **(C)** and the middle of said concave curve segment **(s₁),** said emission surface **(8)** being created either by rotating one of the concave curve segments **(s₁)** around the axis of symmetry **(S)** or by translating both curve segments **(s₁)** in a direction **(X)** perpendicular to the profile plane **(Pₚ)** containing said curve segments, **characterized in that**:
- within the profile plane **(Pₚ),** the two concave curve segments (s₁) follow arcs of first **(E₁)** and second **(E₂)** non-coincident circles that intersect each other, one of the concave curve segments **(s₁)** following the arc of the first circle, that arc being situated within the second circle, while the other of the two concave curve segments **(s₁)** follows the arc of the second circle, the said arc being situated inside the first circle,
- each curve segment **(s₁)** has an acoustic axis **(a)** intersecting the axis of symmetry **(S)** or the plane of symmetry **(A₁)** between the first focal area **(Z₁)** and the emission surface **(8),** the acoustic axes **(a)** being separated from each other, at the first focal area **(Z₁),** by a separating distance **(Dₑ)** comprised between 1 and 50 mm, such that the beams of the emission surface **(8)** intersect to create a second focal area **(Z₂)** that is located and situated between the first focal area **(Z₁)** and the emission surface **(8),** and separated from the focal plane **(Pf),**
- the emission surface **(8)** includes an inner edge **(8₁)** whereof the symmetrical parts extending on either side of the axis of symmetry **(S)** or plane of symmetry **(A₁)** are separated from each other by an inner distance **(Dᵢ)** comprised between 10 and 120 mm allowing the second focal area to be positioned away from the emission surface.

2. The therapeutic probe according to claim 1, **characterized in that** the emission surface **(8)** includes an outer edge whereof the symmetrical parts extending on either side of the axis of symmetry [or] plane of symmetry are separated from each other by an outer distance **(Dₛ)** comprised between 30 and 300 mm.

3. The therapeutic probe according to claim 1, **characterized in that** the inner edge **(8₁)** delimits, in the emission surface **(8),** a housing for mounting an ultrasonic imaging probe.

4. The therapeutic probe according to one of claims 1 to 3, **characterized in that** each concave curve segment **(s₁)** is a concave arc of circle segment.

5. The therapeutic probe according to one of claims 1 to 4, **characterized in that** the emission surface **(8)** results from a toroid geometry created by rotating one of the concave curve segments **(s₁)** around the axis of symmetry **(S)** such that the first focal area **(Z₁)** has a crown shape.

6. The therapeutic probe according to claim 5, **characterized in that** the emission surface **(8)** is truncated symmetrically relative to the axis of symmetry **(S).**

7. The therapeutic probe according to one of claims 1 to 4, **characterized in that** the emission surface **(8)** results from a cylindrical geometry created by translating both curve segments over a finite length in a direction **(X)** perpendicular to the profile plane **(Pp)** containing said curve segments, such that the first focal area **(Z₁)** has a dual linear shape.

8. A therapeutic apparatus for treating tissue by emitting focused ultrasonic waves, including a therapeutic probe **(1)** according to one of claims 1 to 7 whereof the ultrasonic sources **(3)** are activated by signals delivered by a signal generator that is part of a control circuit **(7),** so as to focus the ultrasonic waves in a first focal area **(Z₁)** and obtain a second focal area **(Z₂)** located and situated between the first focal area **(Z₁)** and the emission surface **(8).**
